(19) Europäisches Patentamt
European Patent Office
Office européen des brevets

(11) Publication number: **0 485 026 A2**

(12) **EUROPEAN PATENT APPLICATION**

(21) Application number: **91202828.9**

(22) Date of filing: **31.10.91**

(51) Int. Cl.$^5$: **A61K 31/66**, C07F 9/38

(30) Priority: **08.11.90 GB 9024345**

(43) Date of publication of application:
**13.05.92 Bulletin 92/20**

(84) Designated Contracting States:
**CH DE FR GB IT LI NL**

(71) Applicant: **MERCK SHARP & DOHME LTD.**
**Hertford Road**
**Hoddesdon Hertfordshire EN11 9BU(GB)**

(72) Inventor: **Fletcher, Stephen Robert**
**141 East Park**
**Old Harlow, Essex(GB)**
Inventor: **Kulagowski, Janusz J.**
**24 Elmbrook Drive**
**Bishops Stortford, Hertfordshire(GB)**
Inventor: **Teall, Martin Richard**
**134 Station Road**
**Standon, Near Ware, Hertfordshire(GB)**

(74) Representative: **Thompson, John Dr. et al**
**Merck & Co., Inc. European Patent**
**Department Terlings Park Eastwick Road**
**Harlow, Essex CM20 2OR(GB)**

(54) **Antimanic phenoxymethylene bisphosphonate derivatives.**

(57) A class of substituted phenoxymethylene bisphosphonate derivatives inhibit the action of inositol monophosphatase enzymes and are therefore useful in the treatment and/or prevention of bipolar and nonbipolar depressive disorders, and psychotic disorders other than mania, without exhibiting the spectrum of severe side-effects customarily associated with lithium treatment.

EP 0 485 026 A2

The present invention relates to a class of substituted phenoxymethylene bisphosphonate derivatives which inhibit the action of inositol monophosphatase enzymes and are therefore useful in the treatment and/or prophylaxis of bipolar and nonbipolar depressive disorders, and psychotic disorders other than mania.

A class of substituted methylene bisphosphonate derivatives is described in WO-A-86/00902. These compounds are stated to be capable of maintaining calcium balance within bones and teeth, and therefore to be of use in the treatment or prevention of inter alia osteoporosis, arthritic disorders, atherosclerosis, hypercalcaemia, Paget's disease and dental calculus. There is, however, no suggestion in WO-A-86/00902 that the compounds disclosed therein may be of any use in the treatment of depressive disorders.

Following the discovery of the antimanic properties of lithium by John Cade in 1949, little further progress has been made in this field such that, to date, lithium remains essentially the sole effective treatment for manic depression. Lithium is highly specific in its alleviation of manic symptoms, normalising the mood of manic patients rather than compensating for the excesses of the manic state via sedation or "tranquillisation". Moreover, lithium is perhaps the only drug in psychiatry for which clear prophylaxis against disease recurrences and deterioration has been demonstrated. Lithium is clearly effective in the treatment of bipolar disorders, including both mania and depression, and has also been implicated as a treatment for nonbipolar depressive illness and even psychotic disorders other than mania.

Hallcher and Sherman (J. Biol. Chem., 1980, 255, 10896) demonstrated a profound elevation of inositol-1-phosphate concentration and a corresponding decrease in the concentration of free inositol in the brains of rats treated systemically with lithium, and this effect was attributed to inhibition of inositol monophosphatase.

Despite its remarkable and undisputed therapeutic properties lithium does, however, exhibit a significant number of undesirable side-effects, as discussed in Meyler's Side Effects of Drugs, ed. M.N.G. Dukes, Tenth Edition, 1984, Chapter 3. Furthermore, the antimanic effect of lithium is not manifested until 7 to 10 days after treatment, which means that it may be necessary to administer antipsychotic drugs in the interim to patients suffering from acute bipolar disorder.

These side-effects, and particularly the more serious ones, can be reduced by monitoring plasma lithium concentrations in bipolar patients. However, the need to monitor plasma drug concentrations, and to maintain these within a narrow therapeutic range, clearly detracts from the clinical utility of lithium.

We have found a class of compounds which inhibit the action of inositol monophosphatase enzymes, but which lack the spectrum of severe side-effects customarily associated with lithium.

The present invention provides the use of a compound of formula I, or a pharmaceutically acceptable salt or a prodrug thereof:

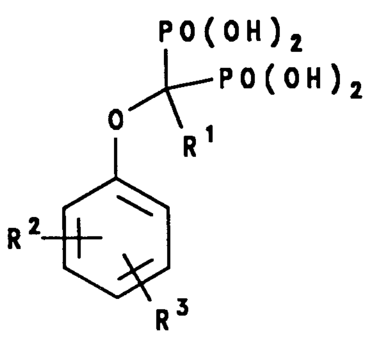

(I)

wherein

$R^1$ represents hydrocarbon;

$R^2$ and $R^3$ independently represent hydrogen, hydrocarbon, halogen, cyano, trifluoromethyl, nitro, $-OR^a$, $-OCOR^a$, $-SR^a$, $-SO_2NR^aR^b$, $-NR^aR^b$, $-NR^aCOR^b$, $-NR^aCO_2R^b$, $-NR^aSO_2R^b$, $-COR^a$, $-CO_2R^a$ or $-CONR^aR^b$, or $R^2$ and $R^3$ together represent methylenedioxy; and

$R^a$ and $R^b$ independently represent hydrogen or hydrocarbon;

for the manufacture of a medicament for the treatment and/or prevention of bipolar and nonbipolar depressive disorders, and psychotic disorders other than mania.

The class of compounds generically disclosed in WO-A-86/00902 corresponds inter alia to the compounds of formula I above wherein R[1] stands for unsubstituted $C_{1-8}$ alkyl or unsubstituted aryl($C_{1-4}$)-alkyl; and the phenoxy moiety is optionally substituted with straight or branched $C_{1-4}$ alkyl, amino, $C_{1-4}$ alkylamino, di($C_{1-4}$)alkylamino, carboxy, $C_{1-4}$ alkoxycarbonyl, hydroxy, $C_{1-4}$ alkoxy, phenoxy, mercapto, $C_{1-4}$ alkylthio, phenylthio, halogen or trifluoromethyl. However, no compound is specifically disclosed in WO-A-86/00902 wherein, when R[1] in formula I above represents a hydrocarbon group, the phenoxy moiety is other than unsubstituted.

The term "hydrocarbon" as used herein includes straight-chained, branched and cyclic groups, including heterocyclic groups, containing up to 25 carbon atoms, suitably up to 18 carbon atoms, and conveniently up to 12 carbon atoms. Suitable hydrocarbon groups include $C_{1-8}$ alkyl, $C_{2-6}$ alkenyl, $C_{2-6}$ alkynyl, $C_{3-7}$ cycloalkyl, $C_{3-7}$ cycloalkyl($C_{1-8}$)alkyl, aryl, aryl($C_{1-8}$)alkyl, $C_{3-7}$ heterocycloalkyl, $C_{3-7}$ heterocycloalkyl($C_{1-8}$)alkyl, heteroaryl and heteroaryl($C_{1-8}$)alkyl.

Suitable alkyl groups include straight-chained and branched alkyl groups containing from 1 to 8 carbon atoms. Typical examples include methyl and ethyl groups, and straight-chained or branched propyl and butyl groups. Particular alkyl groups are n-propyl, isopropyl, n-butyl and t-butyl.

Suitable alkenyl groups include straight-chained and branched alkenyl groups containing from 2 to 6 carbon atoms. Typical examples include vinyl, allyl and dimethylallyl groups.

Suitable alkynyl groups include straight-chained and branched alkynyl groups containing from 2 to 6 carbon atoms. Typical examples include ethynyl and propargyl groups.

Suitable cycloalkyl groups include groups containing from 3 to 7 carbon atoms. Particular cycloalkyl groups are cyclopropyl and cyclohexyl.

Suitable aryl groups include phenyl, naphthyl and tetrahydronaphthyl groups.

Particular aryl($C_{1-8}$)alkyl groups are benzyl, phenethyl, phenylpropyl, phenylpentyl and phenylhexyl.

Suitable heterocycloalkyl groups include pyrrolidinyl, piperidyl, piperazinyl and morpholinyl groups.

Suitable heteroaryl groups include pyridyl, quinolyl, isoquinolyl, pyridazinyl, pyrimidinyl, pyrazinyl, pyranyl, furyl, benzofuryl, thienyl, benzthienyl, indolyl, imidazolyl, benzimidazolyl, thiazolyl, oxadiazolyl and thiadiazolyl groups. Particular heteroaryl groups are pyridyl and furyl.

The hydrocarbon group may in turn be optionally substituted by one or more groups selected from $C_{1-6}$ alkyl, adamantyl, phenyl, halogen, $C_{1-6}$ haloalkyl, trifluoromethyl, hydroxy, $C_{1-6}$ alkoxy, aryloxy, keto, $C_{1-3}$ alkylenedioxy, nitro, cyano, carboxy, $C_{2-6}$ alkoxycarbonyl, $C_{2-6}$ alkoxycarbonyl($C_{1-6}$)alkyl, $C_{2-6}$ alkylcarbonyloxy, optionally substituted arylcarbonyloxy, $C_{2-6}$ alkylcarbonyl, optionally substituted arylcarbonyl, $C_{1-6}$ alkylthio, $C_{1-6}$ alkylsulphinyl, $C_{1-6}$ alkylsulphonyl, amino, mono- or di($C_{1-6}$)alkylamino, $C_{2-6}$ alkylcarbonylamino, optionally substituted arylcarbonylamino, $C_{2-6}$ alkoxycarbonylamino, $C_{1-6}$ aminoalkyl and optionally substituted arylcarbonylamino($C_{1-6}$)alkyl.

The term "halogen" as used herein includes fluorine, chlorine, bromine and iodine, especially chlorine.

Suitable values for the substituent R[1] in the compounds of formula I above include $C_{1-8}$ alkyl and aryl-($C_{1-8}$)alkyl, either of which may be optionally substituted.

Suitably, at least one of R[2] and R[3] represents hydroxy.

Certain compounds falling within the definition of formula I are novel. Accordingly, in a further aspect the present invention provides a compound of formula II, or a salt or prodrug thereof:

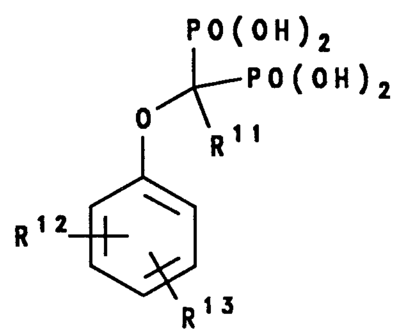

(II)

wherein

$R^{11}$ represents hydrocarbon;

$R^{12}$ and $R^{13}$ independently represent hydrogen, hydrocarbon, halogen, cyano, trifluoromethyl, nitro, $-OR^a$, $-OCOR^a$, $-SR^a$, $-SO_2NR^aR^b$, $-NR^aR^b$, $-NR^aCOR^b$, $-NR^aCO_2R^b$, $-NR^aSO_2R^b$, $-COR^a$, $-CO_2R^a$ or $-CONR^aR^b$, or $R^{12}$ and $R^{13}$ together represent methylenedioxy; and

$R^a$ and $R^b$ independently represent hydrogen or hydrocarbon;

provided that when $R^{11}$ represents unsubstituted $C_{1-8}$ alkyl or unsubstituted aryl($C_{1-4}$)alkyl and $R^{12}$ represents hydrogen, then $R^{13}$ does not represent hydrogen, straight or branched $C_{1-4}$ alkyl, amino, $C_{1-4}$ alkylamino, di($C_{1-4}$)alkylamino, carboxy, $C_{1-4}$ alkoxycarbonyl, hydroxy, $C_{1-4}$ alkoxy, phenoxy, mercapto, $C_{1-4}$ alkylthio, phenylthio, halogen or trifluoromethyl.

The present invention also includes within its scope compounds of formula II above wherein $R^{11}$, $R^{12}$ and $R^{13}$ are as defined with reference to formula II, provided that when $R^{11}$ represents unsubstituted $C_{1-8}$ alkyl or unsubstituted aryl($C_{1-4}$)alkyl, then neither $R^{12}$ nor $R^{13}$ represents hydrogen, straight or branched $C_{1-4}$ alkyl, amino, $C_{1-4}$ alkylamino, di($C_{1-4}$)alkylamino, carboxy, $C_{1-4}$ alkoxycarbonyl, hydroxy, $C_{1-4}$ alkoxy, phenoxy, mercapto, $C_{1-4}$ alkylthio, phenylthio, halogen or trifluoromethyl.

The present invention further includes within its scope compounds of formula II above wherein $R^{11}$, $R^{12}$ and $R^{13}$ are as defined with reference to formula II, provided that $R^{11}$ does not represent unsubstituted $C_{1-8}$ alkyl or unsubstituted aryl($C_{1-4}$)alkyl.

For use in medicine, the salts of the compounds of formula II will be non-toxic pharmaceutically acceptable salts. Other salts may, however, be useful in the preparation of the compounds according to the invention or of their non-toxic pharmaceutically acceptable salts.

Suitable pharmaceutically acceptable salts of the compounds of formulae I and II above include alkali metal (e.g. sodium or potassium) salts; alkaline earth metal (e.g. calcium or magnesium) salts; other metal salts, e.g. aluminium salts; and salts formed with suitable organic ligands, e.g. quaternary ammonium salts. Where they are capable of existing, acid addition salts may, for example, be formed by mixing a solution of the compound with a solution of a pharmaceutically acceptable non-toxic acid such as hydrochloric acid, fumaric acid, maleic acid, succinic acid, acetic acid, citric acid, tartaric acid, carbonic acid or phosphoric acid.

If any side-chain in the compounds of formulae I and II above possesses an asymmetric carbon atom, the resulting compound may accordingly exist as enantiomers. If two or more asymmetric centres are present, diastereoisomers may also exist. It is to be understood that all such stereoisomers and mixtures thereof are encompassed within the scope of the present invention.

The present invention includes within its scope prodrugs of the compounds of formulae I and II above. In general, such prodrugs will be functional derivatives of the compounds concerned which are readily convertible in vivo into the required compound. Conventional procedures for the selection and preparation of suitable prodrug derivatives are described, for example, in "Design of Prodrugs", ed. H. Bundgaard, Elsevier, 1985. In the present case, the compounds of interest are tetrabasic phosphorus acids, and one particular class of prodrugs will accordingly comprise in vivo hydrolysable mono-, di-, tri- or tetraesters thereof.

Suitable values for the in vivo hydrolysable ester residue include the following groups of part formulae (i) to (iii):

$-O.CHR^5.O.CO.R^t$ (i)

$-O.CH_2.CH_2.NR^uR^v$ (ii)

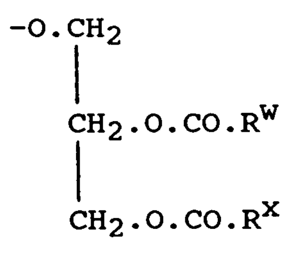

(iii)

wherein $R^s$, $R^t$, $R^u$ and $R^v$ independently represent hydrogen, straight-chained or branched $C_{1-6}$ alkyl, or $C_{3-7}$ cycloalkyl; or $R^u$ and $R^v$ together with the intervening nitrogen atom represent pyrrolidino, piperidino or morpholino; and -CO.$R^w$ and -CO.$R^x$ independently represent a fatty acyl residue such as arachidonyl, palmitoyl, stearoyl or oleoyl. Suitably $R^s$ represents hydrogen and $R^t$ represents methyl or t-butyl; or $R^s$ represents methyl and $R^t$ represents t-butyl. Suitably $R^u$ represents methyl and $R^v$ represents hydrogen or methyl. In particular, it is preferred that -CO.$R^w$ and -CO.$R^x$ each represents arachidonyl.

A particular sub-class of compounds within the scope of the present invention is represented by the compounds of formula III, and salts and prodrugs thereof:

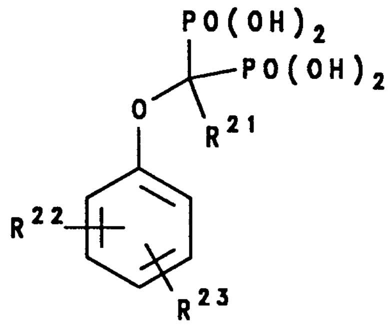

(III)

wherein

$R^{21}$ represents substituted $C_{1-8}$ alkyl, substituted aryl($C_{1-4}$)alkyl, optionally substituted aryl($C_{5-8}$)alkyl, optionally substituted $C_{3-7}$ cycloalkyl($C_{1-8}$)alkyl, optionally substituted $C_{3-7}$ heterocycloalkyl($C_{1-8}$)alkyl or optionally substituted heteroaryl($C_{1-8}$)alkyl;

$R^{22}$ and $R^{23}$ independently represent hydrogen, hydrocarbon, halogen, cyano, trifluoromethyl, nitro, -$OR^a$, -$OCOR^a$, -$SR^a$, -$SO_2NR^aR^b$, -$NR^aR^b$, -$NR^aCOR^b$, -$NR^aCO_2R^b$, -$NR^aSO_2R^b$, -$COR^a$, -$CO_2R^a$ or -$CONR^aR^b$, or $R^{22}$ and $R^{23}$ together represent methylenedioxy; and

$R^a$ and $R^b$ independently represent hydrogen or hydrocarbon.

Representative compounds of formula III above include those compounds wherein $R^{21}$ represents substituted $C_{1-8}$ alkyl, substituted aryl($C_{1-4}$)alkyl or optionally substituted aryl($C_{5-8}$)alkyl; and $R^{22}$ and $R^{23}$ are as defined above.

Suitable values of $R^{21}$ in the compounds of formula III above include substituted methyl, ethyl, propyl, butyl, pentyl, hexyl, benzyl, phenethyl or phenylpropyl, and optionally substituted phenylpentyl or phenylhexyl.

Preferred substituents on the group $R^{21}$ include halogen, trifluoromethyl, hydroxy, $C_{1-4}$ alkoxy, phenoxy, tetrahydronaphthyloxy, nitro, cyano, carboxy, amino, aminomethyl, phenylcarbonylaminomethyl and (dichlorophenyl)carbonylaminomethyl.

Particular values of the group $R^{21}$ include methoxy-methyl, phenoxy-propyl, (phenoxyphenyl)-propyl, amino-propyl, hydroxy-butyl, amino-pentyl, (hydroxyphenyl)pentyl, hydroxy-hexyl, (hydroxyphenyl)hexyl, fluorobenzyl, chlorobenzyl, iodobenzyl, dichlorobenzyl, trifluoromethyl-benzyl, hydroxy-benzyl, phenoxy-benzyl, tetrahydronaphthyloxy-benzyl, nitrobenzyl, cyano-benzyl, carboxy-benzyl, aminomethyl-benzyl, (benzamidomethyl)-benzyl and (dichlorobenzamidomethyl)benzyl.

Suitably, $R^{22}$ and $R^{23}$ independently represent hydrogen, optionally substituted $C_{1-8}$ alkyl, optionally substituted aryl($C_{1-8}$)alkyl, halogen, trifluoromethyl or hydroxy. Preferably, $R^{22}$ and $R^{23}$ independently represent hydrogen or hydroxy. In a particularly preferred embodiment, one of $R^{22}$ and $R^{23}$ represents hydroxy in the 4-position of the phenoxy moiety.

A further sub-class of compounds within the scope of the present invention is represented by the compounds of formula IV, and salts and prodrugs thereof:

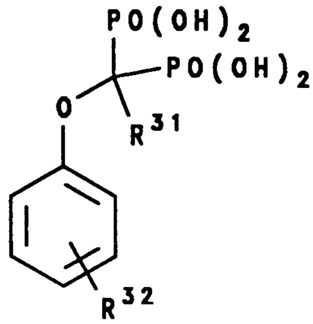

( IV )

wherein

$R^{31}$ represents hydrocarbon;

$R^{32}$ represents hydroxy($C_{1-8}$)alkyl, cyano, nitro, $-OCOR^a$, $-SO_2NR^aR^b$, $-NR^aCOR^b$, $-NR^aCO_2R^b$, $-NR^aSO_2R^b$, $-COR^a$ or $-CONR^aR^b$; and

$R^a$ and $R^b$ independently represent hydrogen or hydrocarbon.

Representative compounds of formula IV above include those compounds wherein $R^{31}$ represents $C_{1-8}$ alkyl or aryl($C_{1-8}$)alkyl, either of which may be optionally substituted; and $R^{32}$ is as defined above.

Preferably, $R^{32}$ represents hydroxymethyl or $-NR^aSO_2R^b$ wherein $R^a$ represents hydrogen or $C_{1-4}$ alkyl,and $R^b$ represents $C_{1-8}$ alkyl or aryl. In a particularly preferred embodiment, $R^{32}$ represents hydroxymethyl, methylsulphonylamino or phenylsulphonylamino.

Specific compounds within the scope of the present invention include:

1-(4-hydroxyphenoxy)-2-methoxyethane-1,1-bisphosphonic acid;
2-(3,4-dichlorophenyl)-1-phenoxyethane-1,1-bisphosphonic acid;
2-(2,4-dichlorophenyl)-1-phenoxyethane-1,1-bisphosphonic acid;
1-(4-hydroxyphenoxy)-4-phenoxybutane-1,1-bisphosphonic acid;
1-(4-hydroxyphenoxy)-4-(4-phenoxyphenyl)butane-1,1-bisphosphonic acid;
1-(4-hydroxyphenoxy)-7-(2-hydroxyphenyl)heptane-1,1-bisphosphonic acid;
1-(4-hydroxyphenoxy)-6-(2-hydroxyphenyl)hexane-1,1-bisphosphonic acid;
5-hydroxy-1-(4-hydroxyphenoxy)pentane-1,1-bisphosphonic acid;
2-(4-cyanophenyl)-1-(4-hydroxyphenoxy)ethane-1,1-bisphosphonic acid;
2-(2-aminomethylphenyl)-1-(4-hydroxyphenoxy)ethane-1,1-bisphosphonic acid;
2-(3-aminomethylphenyl)-1-(4-hydroxyphenoxy)ethane-1,1-bisphosphonic acid;
2-(4-aminomethylphenyl)-1-(4-hydroxyphenoxy)ethane-1,1-bisphosphonic acid;
6-amino-1-(4-hydroxyphenoxy)hexane-1,1-bisphosphonic acid;
2-(3-benzamidomethylphenyl)-1-(4-hydroxyphenoxy)ethane-1,1-bisphosphonic acid;
1-(4-methanesulphonamidophenoxy)ethane-1,1-bisphosphonic acid;
1-(4-hydroxyphenoxy)-2-(2-hydroxyphenyl)ethane-1,1-bisphosphonic acid;
1-(4-hydroxyphenoxy)-2-(4-hydroxyphenyl)ethane-1,1-bisphosphonic acid;
7-hydroxy-1-(4-hydroxyphenoxy)heptane-1,1-bisphosphonic acid;
1-(4-hydroxyphenoxy)-2-(4-methylphenyl)ethane-1,1-bisphosphonic acid;
1-(4-hydroxyphenoxy)-2-(4-phenoxyphenyl)ethane-1,1-bisphosphonic acid;
1-(4-hydroxyphenoxy)-2-(4-iodophenyl)ethane-1,1-bisphosphonic acid;
1-(4-hydroxyphenoxy)-2-(3-trifluoromethylphenyl)ethane-1,1-bisphosphonic acid;
2-(3,4-dichlorophenyl)-1-(4-hydroxyphenoxy)ethane-1,1-bisphosphonic acid;
1-(4-hydroxyphenoxy)-2-[4-(5,6,7,8-tetrahydronaphth-1-yloxy)phenyl]ethane-1,1-bisphosphonic acid;
4-amino-1-(4-hydroxyphenoxy)butyl-1,1-bisphosphonic acid;
2-(3-cyanophenyl)-1-phenoxyethane-1,1-bisphosphonic acid;
2-(4-chlorophenyl)-1-phenoxyethane-1,1-bisphosphonic acid;
2-(3-carboxyphenyl)-1-phenoxyethane-1,1-bisphosphonic acid;
2-(4-fluorophenyl)-1-phenoxyethane-1,1-bisphosphonic acid;
2-(3-nitrophenyl)-1-phenoxyethane-1,1-bisphosphonic acid;
2-[4-(3,4-dichlorobenzamidomethyl)phenyl]-1-(4-hydroxyphenoxy)ethane-1,1-bisphosphonic acid;

2-[3-(3,4-dichlorobenzamidomethyl)phenyl]-1-(4-hydroxyphenoxy)ethane-1,1-bisphosphonic acid;
1-[3-(hydroxymethyl)phenoxy]ethane-1,1-bisphosphonic acid;
and salts and prodrugs thereof.

Further novel compounds according to the present invention include the following:
1-(4-hydroxyphenoxy)ethane-1,1-bisphosphonic acid;
1-(4-hydroxyphenoxy)butane-1,1-bisphosphonic acid;
1-(4-hydroxyphenoxy)-2-phenylethane-1,1-bisphosphonic acid;
1-(4-hydroxyphenoxy)-4-phenylbutane-1,1-bisphosphonic acid;
1-(4-hydroxyphenoxy)-4-methylpentane-1,1-bisphosphonic acid;
1-(2,4-dihydroxyphenoxy)ethane-1,1-bisphosphonic acid;
1-(3,4-dihydroxyphenoxy)ethane-1,1-bisphosphonic acid;
1-[(4-hydroxy-2-n-propyl)phenoxy]ethane-1,1-bisphosphonic acid;
1-[(4-hydroxy-3-methyl)phenoxy]ethane-1,1-bisphosphonic acid;
1-(3-hydroxyphenoxy)ethane-1,1-bisphosphonic acid;
1-(4-aminophenoxy)ethane-1,1-bisphosphonic acid;
and salts and prodrugs thereof; which also includes 1-(4-hydroxyphenoxy)ethane-1,1-bisphosphonic acid, tetrapivaloyloxymethyl ester;
and salts thereof.

The invention also provides pharmaceutical compositions comprising one or more of the novel compounds according to the invention in association with a pharmaceutically acceptable carrier. Preferably these compositions are in unit dosage forms such as tablets, pills, capsules, powders, granules, sterile parenteral solutions or suspensions, or suppositories, for oral, parenteral or rectal administration. For preparing solid compositions such as tablets, the principal active ingredient is mixed with a pharmaceutical carrier, e.g. conventional tableting ingredients such as corn starch, lactose, sucrose, sorbitol, talc, stearic acid, magnesium stearate, dicalcium phosphate or gums, and other pharmaceutical diluents, e.g. water, to form a solid preformulation composition containing a homogeneous mixture of a compound of the present invention, or a non-toxic pharmaceutically acceptable salt thereof. When referring to these preformulation compositions as homogeneous, it is meant that the active ingredient is dispersed evenly throughout the composition so that the composition may be readily subdivided into equally effective unit dosage forms such as tablets, pills and capsules. This solid preformulation composition is then subdivided into unit dosage forms of the type described above containing from 0.1 to about 500 mg of the active ingredient of the present invention. The tablets or pills of the novel composition can be coated or otherwise compounded to provide a dosage form affording the advantage of prolonged action. For example, the tablet or pill can comprise an inner dosage and an outer dosage component, the latter being in the form of an envelope over the former. The two components can be separated by an enteric layer which serves to resist disintegration in the stomach and permits the inner component to pass intact into the duodenum or to be delayed in release. A variety of materials can be used for such enteric layers or coatings, such materials including a number of polymeric acids and mixtures of polymeric acids with such materials as shellac, cetyl alcohol and cellulose acetate.

The liquid forms in which the novel compositions of the present invention may be incorporated for administration orally or by injection include aqueous solutions, suitably flavoured syrups, aqueous or oil suspensions, and flavoured emulsions with edible oils such as cottonseed oil, sesame oil, coconut oil or peanut oil, as well as elixirs and similar pharmaceutical vehicles. Suitable dispersing or suspending agents for aqueous suspensions include synthetic and natural gums such as tragacanth, acacia, alginate, dextran, sodium carboxymethylcellulose, methylcellulose, polyvinylpyrrolidone or gelatin.

In the treatment of depressive disorders, a suitable dosage level is about 0.01 to 250 mg/kg per day, preferably about 0.05 to 100 mg/kg per day, and especially about 0.05 to 5 mg/kg per day. The compounds may be administered on a regimen of 1 to 4 times per day.

The compounds of formula I above, including the novel compounds according to the invention, may be prepared by a process which comprises reacting a compound of formula $R^1$-X with an anion of a compound of formula V:

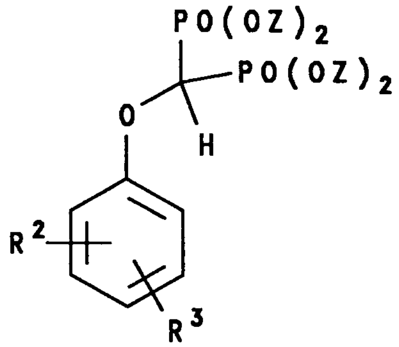

(V)

wherein $R^1$, $R^2$ and $R^3$ are as defined above, X represents a leaving group, and Z represents a suitable protecting group; subsequently removing the protecting groups; and optionally forming a prodrug of the compound thereby obtained.

The reaction is conveniently carried out in a polar aprotic solvent such as N,N-dimethylformamide, ideally at room temperature for a period of 4 to 48 hours, preferably 18 hours. Alternatively, phase transfer catalysis conditions may advantageously be employed.

The anion of the compound of formula V is conveniently prepared by treating the appropriate compound of formula V above with a strong base such as an alkali metal hydride, e.g. sodium hydride. This process may suitably be effected in situ in the reaction mixture.

Suitable values for the leaving group X include halogen, p-toluenesulphonyl and trifluoromethanesulphonyl. Preferably, X represents iodo.

Suitable protecting groups for Z include ester-forming groups such as $C_{1-6}$ alkyl, e.g. methyl or ethyl; $C_{2-6}$ alkenyl, e.g. allyl; and aryl($C_{1-6}$)alkyl, e.g. benzyl. Preferably, Z is ethyl.

Removal of the protecting group Z may be effected by conventional procedures for cleaving phosphonate esters, such as treatment with, for example, concentrated hydrochloric acid, bromotrimethylsilane or iodotrimethylsilane.

The preparation of the compounds of formula V above may be effected by reacting an anion of a compound of formula VI:

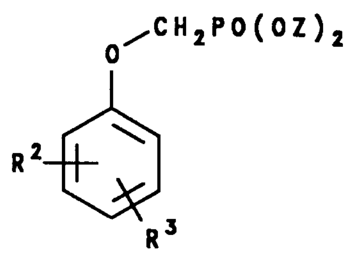

(VI)

wherein $R^2$, $R^3$ and Z are as defined above; with a suitable phosphorylating agent.

The reaction is conveniently carried out in an aprotic solvent such as tetrahydrofuran, ideally at a low temperature, preferably at a temperature below -60 °C.

The anion of the compound of formula VI is suitably prepared by treating the appropriate compound of formula VI above with two equivalents of a strong base, for example lithium diisopropylamide. This process will suitably be effected in situ prior to addition of the phosphorylating agent into the reaction mixture.

The phosphorylating agent employed will preferably be a compound of formula VII:

$A\text{-}PO(OZ)_2$ (VII)

wherein Z is as defined above; and A represents a readily displaceable group, especially chloro.

The intermediates of formula VI above may be prepared by reacting a phenol derivative of formula VIII:

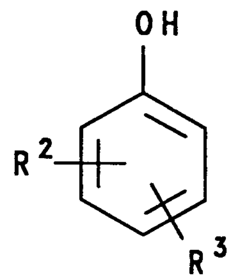

(VIII)

wherein $R^2$ and $R^3$ are as defined above; with a compound of formula IX:

$Y\text{-}CH_2PO(OZ)_2$ (IX)

wherein Z is as defined above, and Y represents a suitable leaving group.

The reaction is conveniently carried out in the presence of a strong base, e.g. sodium hydride or lithium diisopropylamide, in an aprotic solvent such as tetrahydrofuran at a temperature of from 0 to 20°C for a period of from 4 to 48 hours.

The leaving group Y is preferably trifluoromethanesulphonyl, in which case the reagent of formula IX may be prepared by the method described in Tetrahedron Lett., 1986, 27, 1477.

The reagents of formula VIII, where they are not commercially available, may be prepared by the procedures described in the accompanying Examples, or by methods analogous thereto.

As stated previously, the prodrugs of the compounds of formula I above, including the novel compounds according to the invention, may be prepared by standard procedures known from the art. In particular, tetraester derivatives of the compounds of the invention may be prepared by a method analogous to that described in WO-A-86/00902.

It will be appreciated that any compound of formula I initially obtained from the above process may, where appropriate, subsequently be elaborated into a further compound of formula I by techniques known from the art. For example, an O-benzyl ether derivative may be cleaved by standard procedures to the corresponding hydroxy compound; or a cyano group may be converted by reduction into an aminomethyl group, which in turn may subsequently be acylated.

Under certain circumstances, the above-described process may give rise to mixtures of stereoisomers. At an appropriate stage, these isomers may be separated by conventional techniques such as preparative chromatography.

The novel compounds may be prepared in racemic form, or individual enantiomers may be prepared either by enantiospecific synthesis or by resolution. The novel compounds may, for example, be resolved into their component enantiomers by standard techniques, such as the formation of diastereomeric pairs by salt formation with an optically active acid, such as (-)-di-p-toluoyl-d-tartaric acid and/or (+)-di-p-toluoyl-l-tartaric acid followed by fractional crystallization and regeneration of the free base. The novel compounds may also be resolved by formation of diastereomeric esters or amides, followed by chromatographic separation and removal of the chiral auxiliary.

During any of the above synthetic sequences it may be necessary and/or desirable to protect or mask sensitive or reactive groups on any of the molecules concerned. This may be achieved by means of conventional protecting groups, such as those described in Protective Groups in Organic Chemistry, ed.

J.F.W. McOmie, Plenum Press, 1973; and T.W. Greene, Protective Groups in Organic Synthesis, John Wiley & Sons, 1981. The protecting groups may be removed at a convenient subsequent stage using methods known from the art.

The following Examples illustrate the preparation of compounds according to the invention. The inhibitory effect on the enzyme inositol monophosphatase of the compounds of the invention was assayed according to the method described in J. Biol. Chem., 1980, 255, 10896. The concentration of the compounds of accompanying Examples 1 to 15 required to reduce the activity of inositol monophosphatase by 50% ($IC_{50}$) was found to be less than 50 $\mu$M in each case.

## EXAMPLE 1

### 1-(4-Hydroxyphenoxy)-2-methoxyethane-1,1-bisphosphonic acid, biscyclohexylamine salt.

a) Diethyl (4-benzyloxyphenoxy)methylphosphonate; Sodium hydride (80% oil dispersion, 2.3g, 0.077mol) was added portion wise to a solution of 4-benzyloxyphenol (21g, 0.07mol) in dry THF (500mL) stirring at room temperature. After 30 min, diethyl trifluoromethylsulphonyloxymethyl-phosphonate (21g, 0.07mol) was added dropwise such that the reaction temperature did not rise above 20°C. The resulting homogenous solution was stirred at room temperature for 18h, partitioned between ethyl acetate (500mL) and 3N HCl (100mL), the organic phase separated, washed with brine (100mL), dried ($MgSO_4$) and evaporated to afford an oil which was purified by column chromatography on silica with ethyl acetate:hexane 2:1 as eluant to give 21g (86% yield) of product as a colourless oil. $^1H$ NMR (250 MHz, $CDCl_3$) $\delta$; 1.30-1.40 (6H, m), 4.15-4.30 (6H, m), 5.05 (2H, s), 6.90 (4H, s), 7.3-7.5 (5H,m).

b) Tetraethyl (4-benzyloxyphenoxy)methane-1,1-bisphosphonate; A solution of 1a (12g, 0.034mol) in THF (15mL) was added dropwise (10 min) to a freshly prepared solution of LDA (1.6M BuLi in hexanes 48mL, 0.077mol), $iPr_2NH$ (7.8g, 0.077mol), THF (80mL) cooled below -55°C under nitrogen.

After 15 min, diethyl chlorophosphate (6.2g, 0.036mol) was added in one portion. The reaction was warmed to room temperature over 2h, partitioned between ethyl acetate (200mL) and 1N HCl (100mL), the organic phase separated, evaporated in vacuo and the residue purified directly by column chromatography on silica with ethyl acetate -> ethyl acetate:methanol 95:5 as eluant to afford 15g (91% yield) of product as a colourless oil. $^1H$ NMR (250MHz, $CDCl_3$) $\delta$; 1.23-1.36 (12H, m), 4.07-4.34 (8H, m), 4.72 (1H, t,J = 17.45Hz), 5.01 (2H, s), 6.87 and 7.04 (4H, ABq, J = 9.15Hz), 7.26-7.43 (5H,m).

c) Tetraethyl 1-(4-benzyloxyphenoxy)-2-methoxyethane-1,1-bisphosphonate; Sodium hydride (80% oil dispersion, 0.12g, 0.00412mol) was added portionwise to a solution of 1b (1g, 0.00206mol) in dry DMF (5mL) stirring at room temperature. After 30 min, bromomethyl methyl ether (0.34mL, 0.00412mol) was added and the reaction mixture stirred at room temperature for 18h. The mixture was subsequently partitioned between ethyl acetate and water, the organic phase separated, dried ($MgSO_4$) and evaporated to afford an oil which was purified by column chromatography on silica with ethyl acetate -> ethyl acetate:methanol 95:5 as eluant to give 0.45g (41% yield) of product as a colourless oil. $^1H$ NMR (360MHz, $CDCl_3$) $\delta$; 1.3-1.4 (12H), 3.06(3H, s), 3.8-3.9 (2H, dd), 4.14-4.40 (8H, m), 5.04 (2H, s), 6.82-6.90 (2H, m), 7.24-7.46(7H, m).

d) 1-(4-Benzyloxyphenoxyl)-2-methoxyethane-1,1-bisphosphonic acid; A solution of 1c (0.45g, 0.00085mol) in TMS bromide (2.2mL) was stirred at room temperature for 18h. the excess reagent was removed in vacuo, the residue stirred in 10% aqueous acetone for 15min and the solvent then removed in vacuo, replaced by water and the resulting solution lyophilised to afford 0.35g (100% yield) of product as a colourless powder. $^1H$ NMR (250MHz, $D_2O$) $\delta$; 3.10 (3H, s), 3.8-3.95 (3H, t), 5.20 (2H,s), 7.0 and 7.3 (4H, ABq, J = 9Hz), 7.4-7.5 (5H, m).

e) 1-(4-Hydroxyphenoxy)-2-methoxyethane-1,1 bisphosphonic acid; A solution of 1d (0.35g, 0.00084mol) in 1:1 methanol:water (20mL) was shaken on the Parr hydrogenator at 55 psi $H_2$ for 3h with palladium hydroxide on carbon (0.035g) as catalyst. The catalyst was removed by filtration and the solvent evaporated. The residue was dissolved in water, passed down a Dowex 50 X 8-(200 mesh) ($H^+$) ion exchange column (1mL), eluted with water and the acidic, UV active fractions combined and lyophilised to afford 0.28g (100% yield) of product as a colourless powder. $^1H$ NMR (250MHz, $D_2O$) $\delta$; 3.10 (3H, s), 3.80-3.94 (2H, t), 6.83 and 7.21 (4H, ABq, J = 9Hz).

f) 1-(4-Hydroxyphenoxy)-2-methoxyethane-1,1-bisphosphonic acid, biscyclohexylamine salt; A solution of cyclohexylamine (0.3mL, 0.00255mol) in ether (1mL) was added dropwise to a methanolic solution of 1e - (0.28g, 0.00084mol) at room temperature. The resulting precipitate was collected by filtration and dried to afford the biscyclohexylamine salt as a colourless powder. $^1H$ NMR (360MHz, $D_2O$) $\delta$; 1.1-2.1(20H, m), 3.1 (3H, s), 3.0-3.2 (2H, m), 3.85-4.00 (2H, t), 6.8 and 7.3 (4H, ABq, J = 9Hz).

EXAMPLE 2

2-[3,4-Dichlorophenyl]-1-phenoxyethane-1,1-bisphosphonic acid

This compound was prepared as a colourless powder via Steps 1a-d replacing 4-benzyloxyphenol by phenol in Step 1a and bromomethyl methyl ether by 3,4-dichlorobenzyl iodide in Step 1c. $^1$H NMR (360MHz, $D_2O$) δ; 3.44 (2H, t, J = 15Hz). 7.15-7.48 (8H, m).

EXAMPLE 3

1-(4-Hydroxyphenoxy)-4-phenoxybutane-1,1-bisphosphonic acid, tetracyclohexylamine salt.

This compound was prepared as a colourless powder via Steps 1a-f replacing bromomethyl methyl ether by phenoxypropyl iodide in Step 1c. $^1$H NMR (360MHz, $D_2O$) δ; 1.1-2.0 (42H, m), 2.0-2.3 (2H, m), 3.0-3.2 (4H,m), 3.85-3.95 (2H, t), 6.65-7.4 (9H, m).

EXAMPLE 4

1-(4-Hydroxyphenoxy)-4-(4-phenoxyphenyl)butane-1,1-bisphosphonic acid, tetracyclohexylamine salt.

This compound was prepared as a colourless powder via Steps 1a-f replacing bromomethyl methyl ether by 3-(4-phenoxyphenyl)propyl iodide in Step 1c. $^1$H NMR (360MHz, $D_2O$) δ; 1.2-2.2 (44H, m), 2.4-2.5 (2H, t), 3.0-3.2 (4H, m), 6.7 (2H, d, J = 9Hz), 6.9-7.5(11H, m).

EXAMPLE 5

1-(4-Hydroxyphenoxy)-7-(2-hydroxyphenyl)heptane-1,1-bisphosphonic acid.

a) 6-(2-Benzyloxyphenyl)hex-5-yn-1-ol; A mixture of 5-hexyn-1-ol (2.0g, 0.02mol), 1-benzyloxy-2-iodobenzene (8.2g, 0.027mol), cuprous iodide (0.023g, 0.000122mol) and $PdCl_2(PPh_3)_2$ (0.086g, 0.000122mol) in triethylamine (20mL) under nitrogen was stirred at room temperature for 18h. The mixture was then partitioned between ethyl acetate and 1N HCl, the organic phase separated, washed with $NaHCO_3$ solution, dried ($MgSO_4$), and evaporated to afford an oil which was purified by column chromatography on silica with ethyl acetate:hexane 40% -> 100% as eluant to give 4.86g (85% yield) of products as an orange oil $^1$H NMR (250MHz, $CDCl_3$) δ; 1.62-1.78 (4H, m), 2.46-2.54 (2H, t), 3.60-3.66 (2H, t), 5.14 (2H, s), 6.89-6.92 and 7.16-7.50 (9H, m).

b) 6-(2-Benzyloxyphenyl)-1-iodohex-5-yne; A solution of 5a (4.86g, 0.0174 mol), tosyl chloride (4.0g, 0.0208mol), 4-dimethylaminopyridine (0.2g, 0.00174mol) and triethylamine (3.6mL, 0.026mol)in dichloromethane (100mL) was stirred at room temperature for 18h to afford the tosylate which was heated at reflux temperature in acetone with excess sodium iodide for 3h to give 5b as a colourless liquid. $^1$H NMR (250MHz, $CDCl_3$) δ; 1.64-1.78 (2H, m), 1.90-2.04 (2H, m), 2.46-2.54 (2H, t), 3.12-3.20 (2H, t), 5.14 (2H, s), 6.86-6.94 and 7.16-7.52 (9H, m).

c) Tetraethyl 1-(4-benzyloxyphenoxy)-7-(2-benzyloxyphenyl)hept-6-yn-1,1-bisphosphonate; Sodium hydride (80% oil dispersion, 0.12g, 0.00412mol) was added in one portion to a solution of 1b (1g, 0.00206mol) in dry DMF (5mL) stirring at room temperature. After 30 min, 5b (1.6g, 0.00412mol) was added and stirring continued for 18h. The mixture was then partitioned between ethyl acetate and water, the organic phase separated, washed with water, dried ($MgSO_4$) and evaporated to afford an oil which was purified by column chromatography on silica with 75% ethyl acetate:petrol (bp 60-80) an eluant to afford 0.13g (8% yield) of 5c as a yellow oil. $^1$H NMR(360MHz, $CDCl_3$) δ; 1.04-1.62 (4H, m), 1.30-1.40 (12H, t), 1.84-2.10 (2H, m), 3.16-3.24 (2H, t), 4.16-4.40 (8H, m), 5.02 (2H, s), 6.82-7.48 (9H, m).

d) 1-(4-Hydroxyphenoxy)-7-(2-hydroxyphenyl)heptane-1,1-bisphosphonic acid; This compound was prepared as a colourless powder via Steps 1d and 1e replacing 1c by 5c in Step 1d. $^1$H NMR (250MHz, $D_2O$) δ; 0.8-1.4 (8H, m), 1.8-2.4 (2H, m), 2.2-2.4 (2H, m), 6.4-7.15 (8H, m).

EXAMPLE 6

1-(4-Hydroxyphenoxy)-6-(2-hydroxyphenyl)hexane-1,1-bisphosphonic acid.

This compound was prepared as a colourless powder via Steps 5a-d replacing 5-hexyn-1-ol by 4-pentyn-1-ol in Step 5a. $^1$H NMR (250MHz, $CD_3CN$, $D_2O$) δ; 1.1-1.24 (2H, m), 1,32-1.66 (4H, m), 1.88-2.10 (2H, m), 2.45 (2H, t), 6.74-7.28 (8H, m).

EXAMPLE 7

5-Hydroxy-1-(4-hydroxyphenoxy)pentane-1,1-bisphosphonic acid, triscyclohexylamine salt.

This compound was prepared as a colourless powder via Steps 1a-f replacing bromomethyl methyl ether by 4-benzyloxybutyl iodide in Step 1c. $^1$H NMR (360MHz, $D_2O$) δ; 1.1-2.2 (36H, m), 3.0-3.2 (3H, m), 3.5-3.6(2H, m), 6.75 and 7.15 (4H, ABq J = 9Hz).

EXAMPLE 8

2-(4-Cyanophenyl)-1-(4-hydroxyphenoxy)ethane-1,1-bisphosphonic acid, tetracyclohexylamine salt.

This compound was prepared as a colourless powder via Steps 1a-f replacing bromomethyl methyl ether by 4-cyanobenzyl bromide in Step 1c. $^1$H NMR (250MHz, $D_2O$) δ; 1.08-2.4 (40H, m), 3.0-3.3 (4H, m), 3.2 (2H, t), 6.64 (2H, d), 7.0 (2H, d), 7.1 (2H, d), 7.26 (2H, d).

EXAMPLE 9

2-(3-Aminomethylphenyl)-1-(4-hydroxyphenoxyethane-1,1-bisphosphonic acid, biscyclohexylamine salt.

This compound was prepared via Steps 1a-f replacing bromomethyl methyl ether by 3-cyanobenzyl bromide in Step 1c; increasing the hydrogenation time to 18h in Step 1e and omitting the Dowex procedure in Step 1e. $^1$H NMR (250MHz, $D_2O$) δ; 1.06-2.10 (20H, m), 3.06-3.24 (2H, m), 3.4 (2H, t, J = 15Hz), 4.12 (2H, s), 6.78 (2H, d, J = 9Hz), 7.22-7.58 (6H, m).

EXAMPLE 10

6-Amino-1-(4-hydroxyphenoxy)hexane-1,1-bisphosphonic acid triammonium salt.

This compound was prepared as a colourless powder via Steps 1a-e replacing bromomethyl methyl ether by 1-azido-5-iodopentane in Step 1c and omitting the Dowex procedure in Step 1e. Final purification was carried out by reverse-phase HPLC on C-18 silica with 50mM ammonium acetate:acetonitrile 95:5 as eluant. $^1$H NMR (250MHz, $CD_3CN$, $D_2O$) δ; 1.1-1.26 (2H, m), 1.4-1.62 (4H, m), 1.8-2.04 (2H, m), 2.86 (2H, m), 6.82 (2H, d), 7.2 (2H, d).

EXAMPLE 11

2-(3-Benzamidomethylphenyl)-1-(4-hydroxyphenoxyethane)-1,1-bisphosphonic acid.

a) Tetraethyl 1-(4-benzyloxyphenoxy)-2-(3-cyanophenyl)ethane-1,1-bisphosphonate; This compound was prepared as a colourless oil in 44% yield via Step 1c replacing bromomethyl methyl ether by 3-cyanobezyl bromide. $^1$H NMR (360MHz, $CDCl_3$) δ; 1.10-1.18 (6H, t), 1.24-1.32 (6H, t), 3.32-3.48 (2H, t), 5.04 (2H, s), 6.84-7.66 (13H, m).

b) Tetraethyl 2-(3-aminomethylphenyl)-1-(4-hydroxyphenoxy)ethane-1,1-bisphosphonate; A solution of 11a (0.5g, 0.000083mol) in ethanolic HCl ethanol (20mL), concentrated HCl (1mL) was shaken for 6h on the Parr hydrogenator at 55 psi $H_2$ with palladium hydroxide on carbon as catalyst(0.1g). The catalyast was then removed by filtration and the solvent evaporated to afford 0.42g (99% yield) of product as a colourless oil. $^1$H NMR (250MHz, $CDCl_3$) δ; 1.1-1.2 (6H, t), 1.2-1.4 (6H, t), 2.4 (2H, s), 3.4 (2H, t, J = 15Hz), 3.95-4.35 (4H, m), 5.1 (2H, broad s), 6.73 (2H, d, J = 9Hz), 7.1-7.7 (6H, m), 8.3 (1H, broad s).

c) Tetraethyl 2-(3-benzamidomethylphenyl)-1-(4-hydroxyphenoxy)ethane-1,1-bisphosphonate; Benzoyl chloride (0.24mL, 0.00208mL) was added to a solution of 11b (0.42g, 0.00083mol) and triethylamine (0.34mL, 0.00245mol) in dichloromethane (10mL) and DMF (1mL). The resulting solution was stirred at room temperature for 16h, partitioned between ethyl acetate and water the organic phase separated, dried ($MgSO_4$) and evaporated to afford an oil which was purified by column chromatography on silica with ethyl acetate as eluant to give 0.33g (56% yield) of the benzoate ester. This was hydrolysed by stirring in methanolic potassium carbonate (3 equivalents) for 3h at room temperature to afford 0.25g (88% yield) of 11c as a yellow oil. $^1H$ NMR (250MHz, $CDCl_3$) $\delta$; 1.10-1.20 (6H, t), 1.25-1.35 (6H, t), 2.35 (2H, s), 3.35 (2H, t, J = 15Hz), 3.9-4.35 (8H, m), 6.78 (2H, d, J = 9Hz), 7.1-7.9 (11H, m).

d) 2-(3-Benzamidomethylphenyl)-1-(4-hydroxyphenoxy)ethane-1,1-bisphosphonic acid; A solution of 11c (0.25g, 0.0004mol) and TMS bromide (1.1mL) in dichloromethane (2mL) was stirred at room temperature for 16h. The solvent and excess reagent was then evaporated in vacuo and the residue stirred in 50% aqueous acetone (5mL)at room temperature for 20 min. The solvent was evaporated and the residue purified by reverse-phase HPLC on C-18 silica with 20% acetonitrile:50mM ammonium acetate as eluant to give after passage down a Dowex 50X 8-200 mesh ($H^+$) ion exchange column eluted with water 0.07g (35% yield) of product as a colourless powder. $^1H$ NMR (360MHz, $D_2O$) $\delta$; 3.49 (2H, t, 15Hz), 4.52 (2H, s), 6.66 (2H, d, J = 9Hz), 7.16 (2H, d, J = 9Hz), 7.2-7.77 (9H, m).

EXAMPLE 12

1-[4-Methanesulphonamidophenoxy]ethane-1,1-bisphosphonic acid

a) Tetraethyl 1-(4-Nitrophenoxy)ethane-1,1-bisphosphonate; Tetraethyl [4-nitrophenoxy]-methanebisphosphonate (prepared by the method of Binderup, WO Patent 86/00902) was methylated via Step 1c replacing bromomethyl methyl ether by methyl iodide to give 12a as a yellow oil. $^1H$ NMR (250MHz, $CDCl_3$) $\delta$; 1.4 (12H, t, J = 6Hz), 1.65 (3H, t, J = 15Hz), 4.2-4.5 (8H, m), 7.5 and 8.15 (4H, ABq, J = 9Hz).

b) Tetraethyl 1-(4-aminophenoxy)ethane-1,1-bisphosphonate; To a solution of 12a (0.55g, 0.0013mol) in methanol (5mL) at room temperature under nitrogen was added hydrazine hydrate (0.24mL, 0.0052mol) and 10% palladium-on-carbon (0.25g). The resulting slurry was stirred for 2.5h, the catalyst removed by filtration and the solvent evaporated to afford an oil which was purified by column chromatography on silica with dichloromethane:methanol 9:1 as eluant to give 0.33g (66% yield) of product as a colourless solid. $^1H$ NMR (250MHz, $CDCl_3$) $\delta$; 1.35 (12H, t, J = 6Hz), 1.5 (3H, t, J = 15Hz), 3.3 (2H, broads), 4.2-4.4 (8H, m), 6.55 and 7.12 (4H, ABq, J = 9Hz).

c)Tetraethyl 1-(4-methanesulphonamidophenoxy)ethane-1,1-bisphosphonate; To a solution of 12b - (0.08g, 0.0002mol) and triethylamine (0.083mL, 0.0006mol) in THF (3mL) was added methanesulphonyl chloride (0.027mL, 0.00036mol). The mixture was stirred for 4h, washed with water, the organic phase separated, dried ($MgSO_4$) and the solvent evaporated to give an oil which was purified by column chromatography on silica with dichloromethane:methanol 95:5 as eluent to afford 42mg (50% yield) of product as an oil. $^1H$ NMR (250MHz, $CDCl_3$) $\delta$; 1.3-1.45 (12H, m), 1.55 (3H, t. J = 15Hz), 2.95 (3H, s), 4.2-4.4 (8H, m), 7.2 and 7.3 (4H, ABq, J = 9Hz), 8.38 (1H, s).

d) 1-(4-Methanesulphonamidophenoxy)ethane-1,1-bisphosphonic acid; A solution of 12c (0.042g, 0.0001 mol) in TMS bromide(0.25mL) was stirred at room temperature for 16h. the excess reagent was then evaporated, the residue stirred in methanol (1mL) for 1h and the solvent evaporated to give the product as a gummy solid. $^1H$ NMR (250MHz, $CD_3OD$) $\delta$; 5; 1.55 (3H, t, J = 15Hz), 2.9 (3H, s), 7.2 and 7.3 (4H, ABq, J = 9Hz).

EXAMPLE 13

2-[4-(3,4-Dichlorobenzamidomethyl)phenyl]-1-(4-hydroxyphenoxy)ethane-1,1-bisphosphonic acid.

a) Tetraethyl 2-(4-aminomethylphenyl)-1-(4-hydroxyphenoxy)ethane-1,1-bisphosphonate; This compound was prepared via Steps 11a and 11b replacing 3-cyanobenzyl bromide by 4-cyanobenzyl bromide in Step 11a and extending the hydrogenation time to 24h in Step 11b. $^1H$ NMR (250MHz, $CDCl_3$) $\delta$; 1.15-1.35 (12H,m), 3.32 (2H, t, J = 15Hz), 4.00 (2H, s), 3.9-4.3 (8H, m), 6.72 and 7.18 (4H, ABq, J = 9Hz), 7.3 (4H, s).

b)Tetraethyl 2-[4-(3,4-dichlorobenzamidomethyl)phenyl]-1-[4-hydroxyphenoxy]ethane-1,1-bisphosphonate; This compound was prepared as a colourless oil in 55% yield via Step 11c replacing benzoyl chloride by 3,4-dichlorobenzoyl chloride and 11b by 13a. $^1$H NMR (250MHz, $CDCl_3$) δ; 1.10 (6H, t, J = 6Hz), 1.30 (6H, t, J = 6Hz), 3.30 (2H, t, J = 15Hz), 3.9-4.3 (8H, m), 4.5 (2H, d, J = 6Hz), 6.75 (2H, d, J = 9Hz), 6.85 (1H, t, J = 6Hz), 7.1-7.9 (9H, m).

c) 2-(4-[3,4-Dichlorobenzamidomethyl]phenyl]-1-[4-hydroxyphenoxy]ethane-1,1-bisphosphonic acid; A solution of 13b (0.22g, 0.00032mol) in TMS bromide (0.85mL) was stirred at room temperature for 18h. The excess reagent was then evaporated, the residue stirred in methanol (2mL) for 10 min and the solvent removed. The resulting powder was dissolved in water and passed down a Dowex 50X 8-200 ($H^+$) ion exchange column, eluted with water and the UV active fractions lyophilised to afford 0.15g (81% yield) of product as a colourless solid. $^1$H NMR (250MHz, $D_2O$) δ; 2.91 (2H, t, J = 14Hz), 4.04 (2H, s), 6.21 and 6.79 (4H, ABq, J = 9Hz), 6.69 and 6.90 (4H, ABq, J = 9Hz), 7.16 (1H, d, J = 9Hz), 7.3 (1H, dd, J = 9Hz and J =2Hz), 7.54 (1H, J = 2Hz).

EXAMPLE 14

1-[3-Hydroxymethylphenoxy]ethane-1,1-bisphosphonic acid, tetracyclohexylamine salt

a) Tetraethyl 1-[3-[t-butyldimethylsilyloxymethyl]phenoxy] ethane-1,1-bisphosphonate; This compound was prepared as via Steps 1a-c replacing 4-benzyloxyphenol by 3-[t-butyldimethylsilyloxymethyl]phenol in Step 1a and replacing bromomethyl methyl ether by methyl iodide in Step 1c. $^1$H NMR (360MHz, $CDCl_3$) δ; -0.09 and -0.02 (6H, 2s),0.84 (9H, s), 1.26-1.31 (12H, m), 1.47 (3H, t, J = 16Hz), 4.15-4.25 (8H, m), 4.61 (2H,s), 6.95-7.25 (4H, m).

b)Tetraethyl 1-(3-hydroxymethylphenoxy)ethane-1,1- bisphosphonate; To a solution of 14a (0.2g, 0.00037mol) in THF (5mL) was added a solution of tetrabutylammonium fluoride in THF (1M, 1mL). The solution was stirred at room temperature for 2h, the solvent evaporated and the product purified by column chromatography on silica with ethyl acetate:methanol O-10% as eluant to afford 0.09g (58% yield) of product as a colourless oil. $^1$H NMR (250MHz, $CDCl_3$) δ; 1.34-1.40 (12H, m), 1.53 (3H, t, J = 17Hz), 2.91 (1H, s), 4.18-4.39 (8H, m), 4.64 (2H, s), 7.11-7.34 (4H, m).

c)1-(3-Hydroxymethylphenoxy)ethane-1,1-bisphosphonic acid; Tetracyclohexylamine salt was prepared via Step 13c replacing 13b by 14b. $^1$H NMR (250MHz, $D_2O$) δ; 1.0-2.2 (43H, m), 3.0-3.2 (4H, m), 7.1-7.55 (4H, m).

EXAMPLE 15

2-[3-(3,4-Dichlorobenzamidomethyl)phenyl]-1-(4-hydroxyphenoxy)ethane-1,1-bisphosphonic acid

Prepared analogously to the title compound of Example 13, but commencing instead from 3-cyanobenzyl bromide.

EXAMPLE 16

1-(4-Hydroxyphenoxy)ethane-1,1-bisphosphonic acid, Tetrapivaloyloxymethyl ester

a) 1-(4-Benzyloxyphenoxy)ethane-1,1-bisphosphonic acid

This compound was prepared as a colourless powder via Steps 1a-d replacing bromomethyl methyl ether by methyl iodide in Step 1c. $^1$H NMR (250MHz, DMSO) δ 1.3 (3H, t, J = 18Hz), 5.0 (2H, s), 6.9 and 7.2 (4H, ABq, J = 9Hz), 7.3-7.5 (5H, m).

b) 1-(4-Benzyloxyphenoxy)ethane-1,1-bisphosphonic acid, Silver Salt

A solution of silver trifluoroacetate (14.5g, 4eq) in ethanol (20ml) was added portionwise to a solution of 1-(4-benzyloxyphenoxy)ethane-1,1-bisphosphonic acid (6.4g, 1eq) in ethanol (80ml) stirring at room temperature. A white solid formed instantaneously on addition. After 30 min the precipitate was collected by filtration, washed with ether and dried *in vacuo* to give 8g of product as a grey powder.

c) 1-(4-Benzyloxyphenoxy)ethane-1,1-bisphosphonic acid, Tetrapivaloyloxymethyl ester

Iodomethyl pivalate (1.5g) in dry acetonitrile (10ml)was added in one portion to a slurry of the above silver salt (1g) stirring in acetonitrile (40ml) at room temperature maintained in the dark. Tlc (silica, EtAc:Hexane 2:1) after 10 min showed product Rf, 0.9. After 30 min the resulting yellow precipitate was removed by filtration, the solvent evaporated and the residue partitioned between ether and water. The organic phase was separated, dried ($MgSO_4$) and evaporated to give a yellow oil which was purified by column chromatography on silica with EtAc:Hexane 1:1 as eluant to afford the product as a pale yellow oil. $^1H$ NMR (250MHz, $CDCl_3$) $\delta$ 1.2 (36H, s), 1.5 (3H, t, J = 18Hz), 5.0 (2H, s), 5.65-5.9 (8H, m), 6.85 and 7.2 (4H, ABq, J = 9Hz).

d) 1-(4-Hydroxyphenoxy)ethane-1,1-bisphosphonic acid, Tetrapivaloyloxymethyl ester

The above benzyl ether (0.4g) in ethanol (30ml) was hydrogenated at 55 psi hydrogen in the presence of 10% $Pd(OH)_2/C$ (0.1g) for 18h. Tlc (silica, EtAc:Hexane 1:1) then showed product Rf, 0.2. The catalyst was removed by filtration, the solvent evaporated and the residue purified by column chromatography to afford the product as a yellow oil. $^1H$ NMR (250MHz, $CDCl_3$) $\delta$ 1.2 (36H, s), 1.5 (3H, t, J = 18Hz), 4.5 (1H, s), 5.7-5.9 (8H, m), 6.65 and 7.1 (4H, ABq, J = 9Hz). Elemental analysis; Expected for $C_{32}H_{52}O_{16}P_2$, C, 50.92; H, 6.94; Found: C, 50.88; H, 7.15.

EXAMPLES 17-20

a) 1-(4-Benzyloxyphenoxy)ethane-1,1-bisphosphonic acid, Tetra-, Tri-, unsym-Di, sym-Di and monopivaloyloxymethyl esters

Iodomethylpivalate (2.6g) in acetonitrile (10ml) was added in one portion to a slurry of the silver salt of 1-(4-benzyloxyphenoxy)ethane-1,1-bisphosphonic acid(2g- prepared by Steps 16a-b) in acetonitrile (40ml) stirring at room temperature in the dark. After 18h the resulting yellow precipitate was removed by filtration and the solvent evaporated to afford a yellow oil which was separated by reverse-phase HPLC on C-18 silica with 30% acetonitrile:50mM$NH_4OAc$ → acetonitrile as eluant to give the individual esters:
1-(4-Benzyloxyphenoxy)ethane-1,1-bisphosphonic acid, mono-pivaloyloxymethyl ester, ammonium salt;
1-(4-Benzyloxyphenoxy)ethane-1,1-bisphosphonic acid, unsym-Di-pivloyloxymethyl ester, ammonium salt;
1-(4-Benzyloxyphenoxy)ethane-1,1-bisphosphonic acid, sym-Di-pivaloyloxymethyl ester, ammonium salt;
1-(4-Benzyloxyphenoxy)ethane-1,1-bisphosphonic acid, tri-pivaloyloxymethyl ester, ammonium salt;

EXAMPLE 17

1-(4-Hydroxyphenoxy)ethane-1,1-bisphosphonic acid, Mono-pivaloyloxymethyl ester, Ammonium salt

This compound was prepared as a colourless powder from 1-(4-benzyloxyphenoxy)ethane-1,1-bisphosphonic acid, monopivaloyloxymethyl ester, ammonium salt via Step 16d. $^1H$ NMR (360MHz, $D_2O$) $\delta$ 1.2 (9H, s), 1.42 (3H, t, J = 18Hz), 5.6-5.65 (2H, m),6.8 and 7.1 (4H, ABq, J = 9Hz).

EXAMPLE 18

1-(4-Hydroxyphenoxy)ethane-1,1-bisphosphonic acid, unsym-Dipivaloyloxymethyl ester, Ammonium salt

This compound was prepared as a colourless powder from 1-(4-benzyloxyphenoxy)ethane-1,1-bisphosphonic acid, unsymdipivaloyloxymethyl ester, ammonium salt via Step 16d. $^1H$ NMR (250MHz, $D_2O$) $\delta$ 1.2 (9H, s), 1.3 (9H, s), 1.4-1.55 (3H, dd), 5.7-5.85 (4H, m), 6.8 and 7.15 (4H, ABq, J = 9Hz).

EXAMPLE 19

1-(4-Hydroxyphenoxy)ethane-1,1-bisphosphonic acid, sym-Dipivaloyloxymethyl ester, Ammonium salt

This compound was prepared as a colourless powder from 1-(4-benzyloxyphenoxy)ethane-1,1-bisphosphonic acid, symdipivaloyloxymethyl ester, ammonium salt via Step 16d. $^1H$ NMR (360MHz, $D_2O$) δ 1/2 (18H, s), 1.4 (3H, t, J = 18Hz), 5.6-5.7 (4H, m), 6.8 and 7.15 (4H, ABq, J = 9Hz).

EXAMPLE 20

1-(4-Hydroxyphenoxy)ethane-1,1-bisphosphonic acid, Tripivaloyloxymethyl ester, Ammonium salt

This compound was prepared as a colourless powder from 1-(4-benzyloxyphenoxy)ethane-1,1-bisphosphonic acid, tripivaloyloxymethyl ester, ammonium salt via Step 16d. $^1H$ NMR (250MHz, $CDCl_3$) δ 1.2 (27H, s), 1.4 (3H, t, J = 18Hz), 5.6-5.9 (6H, m), 6.8 and 7.15 (4H, ABq, J = 9Hz).

EXAMPLE 21

Tablet Preparation

Tablets containing 1.0, 2.0, 25.0, 26.0, 50.0 and 100.0mg, respectively of the following compounds are prepared as illustrated below:
1(4-Hydroxyphenoxy)-4-(4-phenoxyphenyl)butane-1,1-bisphosphonic acid, tetracyclohexylamine salt 2-[4-(3,4-Dichlorobenzamidomethyl)phenyl]-1-(4-hydroxyphenoxy)ethane-1,1-bisphosphonic acid

| TABLE FOR DOSES CONTAINING FROM 1-25MG OF THE ACTIVE COMPOUND | | | |
|---|---|---|---|
| | Amount-mg | | |
| Active Compound | 1.0 | 2.0 | 25.0 |
| Microcrystalline cellulose | 49.25 | 48.75 | 37.25 |
| Modified food corn starch | 49.25 | 48.75 | 37.25 |
| Magnesium stearate | 0.50 | 0.50 | 0.50 |

| TABLE FOR DOSES CONTAINING FROM 26-100MG OF THE ACTIVE COMPOUND | | | |
|---|---|---|---|
| | Amount-mg | | |
| Active Compound | 26.0 | 50.0 | 100.0 |
| Microcrystalline cellulose | 52.0 | 100.0 | 200.0 |
| Modified food corn starch | 2.21 | 4.25 | 8.5 |
| Magnesium stearate | 0.39 | 0.75 | 1.5 |

All of the active compound, cellulose, and a portion of the corn starch are mixed and granulated to 10% corn starch paste. The resulting granulation is sieved, dried and blended with the remainder of the corn starch and the magnesium stearate. The resulting granulation is then compressed into tablets containing 1.0mg, 2.0mg, 25.0mg, 26.0mg, 50.0mg and 100mg of the active ingredient per tablet.

## Claims

1. The use of a compound of formula I, or a pharmaceutically acceptable salt or a prodrug thereof:

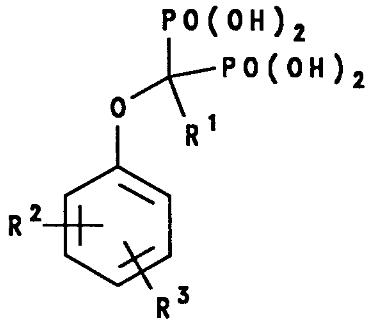

(I)

wherein

$R^1$ represents hydrocarbon;

$R^2$ and $R^3$ independently represent hydrogen, hydrocarbon, halogen, cyano, trifluoromethyl, nitro, $-OR^a$, $-OCOR^a$, $-SR^a$, $-SO_2NR^aR^b$, $-NR^aR^b$, $-NR^aCOR^b$, $-NR^aCO_2R^b$, $-NR^aSO_2R^b$, $-COR^a$, $-CO_2R^a$ or $-CONR^aR^b$, or $R^2$ and $R^3$ together represent methylenedioxy; and

$R^a$ and $R^b$ independently represent hydrogen or hydrocarbon;

for the manufacture of a medicament for the treatment and/or prevention of bipolar and nonbipolar depressive disorders, and psychotic disorders other than mania.

**2.** A compound of formula II, or a salt or prodrug thereof:

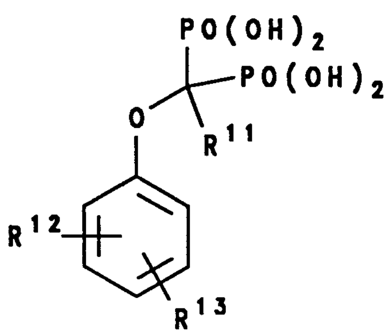

(II)

wherein

$R^{11}$ represents hydrocarbon;

$R^{12}$ and $R^{13}$ independently represent hydrogen, hydrocarbon, halogen, cyano, trifluoromethyl, nitro, $-OR^a$, $-OCOR^a$, $-SR^a$, $-SO_2NR^aR^b$, $-NR^aR^b$, $-NR^aCOR^b$, $-NR^aCO_2R^b$, $-NR^aSO_2R^b$, $-COR^a$, $-CO_2R^a$ or $-CONR^aR^b$, or $R^{12}$ and $R^{13}$ together represent methylenedioxy; and

$R^a$ and $R^b$ independently represent hydrogen or hydrocarbon;

provided that when $R^{11}$ represents unsubstituted $C_{1-8}$ alkyl or unsubstituted aryl($C_{1-4}$)alkyl and $R^{12}$ represents hydrogen, then $R^{13}$ does not represent hydrogen, straight or branched $C_{1-4}$ alkyl, amino, $C_{1-4}$ alkylamino, di($C_{1-4}$)alkylamino, carboxy, $C_{1-4}$ alkoxycarbonyl, hydroxy, $C_{1-4}$ alkoxy, phenoxy, mercapto, $C_{1-4}$ alkylthio, phenylthio, halogen or trifluoromethyl.

**3.** An in vivo hydrolysable mono-, di-, tri-or tetraester of a compound of formula II as defined in claim 2 or a salt thereof.

**4.** A compound as claimed in claim 2 represented by formula III, and salts and prodrugs thereof:

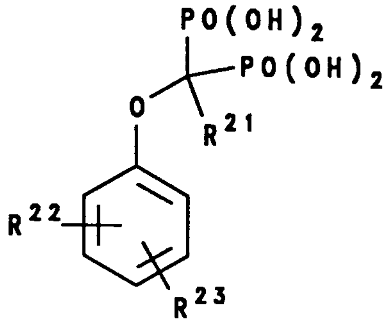

(III)

wherein

$R^{21}$ represents substituted $C_{1-8}$ alkyl, substituted aryl($C_{1-4}$)alkyl, optionally substituted aryl($C_{5-8}$)-alkyl, optionally substituted $C_{3-7}$ cycloalkyl($C_{1-8}$)alkyl, optionally substituted $C_{3-7}$ heterocycloalkyl-($C_{1-8}$)alkyl or optionally substituted heteroaryl($C_{1-8}$)alkyl;

$R^{22}$ and $R^{23}$ independently represent hydrogen, hydrocarbon, halogen, cyano, trifluoromethyl, nitro, $-OR^a$, $-OCOR^a$, $-SR^a$, $-SO_2NR^aR^b$, $-NR^aR^b$, $-NR^aCOR^b$, $-NR^aCO_2R^b$, $-NR^aSO_2R^b$, $-COR^a$, $-CO_2R^a$ or $-CONR^aR^b$, or $R^{22}$ and $R^{23}$ together represent methylenedioxy; and

$R^a$ and $R^b$ independently represent hydrogen or hydrocarbon.

**5.** A compound as claimed in claim 2 represented by formula IV, and salts and prodrugs thereof:

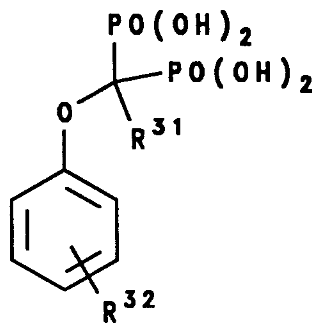

(IV)

wherein

$R^{31}$ represents hydrocarbon;

$R^{32}$ represents hydroxy($C_{1-8}$)alkyl, cyano, nitro, $-OCOR^a$, $-SO_2NR^aR^b$, $-NR^aCOR^b$, $-NR^aCO_2R^b$, $-NR^aSO_2R^b$, $-COR^a$ or $-CONR^aR^b$; and

$R^a$ and $R^b$ independently represent hydrogen or hydrocarbon.

**6.** A compound as claimed in claim 2 selected from:

1-(4-hydroxyphenoxy)-2-methoxyethane-1,1-bisphosphonic acid;
2-(3,4-dichlorophenyl)-1-phenoxyethane-1,1-bisphosphonic acid;
2-(2,4-dichlorophenyl)-1-phenoxyethane-1,1-bisphosphonic acid;
1-(4-hydroxyphenoxy)-4-phenoxybutane-1,1-bisphosphonic acid;
1-(4-hydroxyphenoxy)-4-(4-phenoxyphenyl)butane-1,1-bisphosphonic acid;
1-(4-hydroxyphenoxy)-7-(2-hydroxyphenyl)heptane-1,1-bisphosphonic acid;
1-(4-hydroxyphenoxy)-6-(2-hydroxyphenyl)hexane-1,1-bisphosphonic acid;
5-hydroxy-1-(4-hydroxyphenoxy)pentane-1,1-bisphosphonic acid;

2-(4-cyanophenyl)-1-(4-hydroxyphenoxy)ethane-1,1-bisphosphonic acid;
2-(2-aminomethylphenyl)-1-(4-hydroxyphenoxy)ethane-1,1-bisphosphonic acid;
2-(3-aminomethylphenyl)-1-(4-hydroxyphenoxy)ethane-1,1-bisphosphonic acid;
2-(4-aminomethylphenyl)-1-(4-hydroxyphenoxy)ethane-1,1-bisphosphonic acid;
6-amino-1-(4-hydroxyphenoxy)hexane-1,1-bisphosphonic acid;
2-(3-benzamidomethylphenyl)-1-(4-hydroxyphenoxy)ethane-1,1-bisphosphonic acid;
1-(4-methanesulphonamidophenoxy)ethane-1,1-bisphosphonic acid;
1-(4-hydroxyphenoxy)-2-(2-hydroxyphenyl)ethane-1,1-bisphosphonic acid;
1-(4-hydroxyphenoxy)-2-(4-hydroxyphenyl)ethane-1,1-bisphosphonic acid;
7-hydroxy-1-(4-hydroxyphenoxy)heptane-1,1-bisphosphonic acid;
1-(4-hydroxyphenoxy)-2-(4-methylphenyl)ethane-1,1-bisphosphonic acid;
1-(4-hydroxyphenoxy)-2-(4-phenoxyphenyl)ethane-1,1-bisphosphonic acid;
1-(4-hydroxyphenoxy)-2-(4-iodophenyl)ethane-1,1-bisphosphonic acid;
1-(4-hydroxyphenoxy)-2-(3-trifluoromethylphenyl)ethane-1,1-bisphosphonic acid;
2-(3,4-dichloropheny1)-1-(4-hydroxyphenoxy)ethane-1,1-bisphosphonic acid;
1-(4-hydroxyphenoxy)-2-[4-(5,6,7,8-tetrahydronaphth-1-yloxy)phenyl]ethane-1,1-bisphosphonic acid;
4-amino-1-(4-hydroxyphenoxy)butyl-1,1-bisphosphonic acid;
2-(3-cyanophenyl)-1-phenoxyethane-1,1-bisphosphonic acid;
2-(4-chlorophenyl)-1-phenoxyethane-1,1-bisphosphonic acid;
2-(3-carboxyphenyl)-1-phenoxyethane-1,1-bisphosphonic acid;
2-(4-fluorophenyl)-1-phenoxyethane-1,1-bisphosphonic acid;
2-(3-nitrophenyl)-1-phenoxyethane-1,1-bisphosphonic acid;
2-[4-(3,4-dichlorobenzamidomethyl)phenyl]-1-(4-hydroxyphenoxy)ethane-1,1-bisphosphonic acid;
2-[3-(3,4-dichlorobenzamidomethyl)phenyl]-1-(4-hydroxyphenoxy)ethane-1,1-bisphosphonic acid;
1-[3-(hydroxymethyl)phenoxy]ethane-1,1-bisphosphonic acid;
and salts and prodrugs thereof.

**7.** A compound selected from:
1-(4-hydroxyphenoxy)ethane-1,1-bisphosphonic acid;
1-(4-hydroxyphenoxy)butane-1,1-bisphosphonic acid;
1-(4-hydroxyphenoxy)-2-phenylethane-1,1-bisphosphonic acid;
1-(4-hydroxyphenoxy)-4-phenylbutane-1,1-bisphosphonic acid;
1-(4-hydroxyphenoxy)-4-methylpentane-1,1-bisphosphonic acid;
1-(2,4-dihydroxyphenoxy)ethane-1,1-bisphosphonic acid;
1-(3,4-dihydroxyphenoxy)ethane-1,1-bisphosphonic acid;
1-[(4-hydroxy-2-n-propyl)phenoxy]ethane-1,1-bisphosphonic acid;
1-[(4-hydroxy-3-methyl)phenoxy]ethane-1,1-bisphosphonic acid;
1-(3-hydroxyphenoxy)ethane-1,1-bisphosphonic acid;
1-(4-aminophenoxy)ethane-1,1-bisphosphonic acid;
and salts and prodrugs thereof; which also includes 1-(4-hydroxyphenoxy)ethane-1,1-bisphosphonic acid, tetrapivaloyloxymethyl ester; and salts thereof.

**8.** A compound as claimed in any one of claims 2 to 7 or a pharmaceutically acceptable salt thereof or a prodrug thereof for use in therapy.

**9.** A pharmaceutical composition comprising at least one compound as claimed in any one of claims 2 to 7 or a pharmaceutically acceptable salt thereof or a prodrug thereof in association with one or more pharmaceutically acceptable carriers.

**10.** A process for the preparation of a compound of formula I as defined in claim 1, which process comprises reacting a compound of formula $R^1$-X with an anion of a compound of formula V:

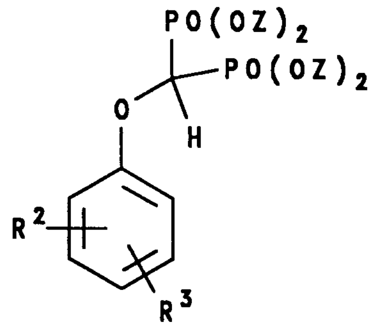

(V)

wherein $R^1$, $R^2$ and $R^3$ are as defined in claim 1, X represents a leaving group, and Z represents a suitable protecting group; subsequently removing the protecting groups; and optionally forming a prodrug of the compound thereby obtained; followed, if desired, by converting a compound of formula I initially obtained into a further compound of formula I using methods known per se.